# DEMANDE DE BREVET EUROPEEN

(11) **EP 1 661 564 A1**
(43) Date de publication de la demande: **31.05.2006**
(21) Numéro de dépôt: 05077194.8
(22) Date de dépôt: 03.11.2000
(51) Int. Cl.: A61K 31/35, A61K 31/40, A61K 31/34, C07D 307/24, A61P 39/06

(54) **Nouveaux composés hétérocycliques et leur application à titre de médicaments**

(30) Priorité: 05.11.1999 FR 9913858; 23.05.2000 FR 0006535
(62) Demande divisionnaire de: 00974646.2
(71) Demandeur: Société de Conseils de Recherches et d'Applications Scientifiques ( S.C.R.A.S.), 75016 Paris (FR)
(72) Inventeur: Auvin, Serge, 91120 Palaiseau (FR); Chabrier de Lassauniere, Pierre Etienne, 75016 Paris (FR)
(74) Mandataire: Bourgouin, André

(57) **Abrégé**

La présente invention concerne de nouveaux dérivés hétérocycliques présentant une activité inhibitrice des calpaïnes et / ou une activité piégeuse des formes réactives de l'oxygène, de formule 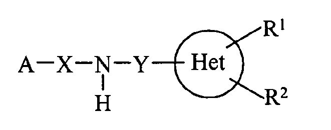 dans laquelle A représente un radical 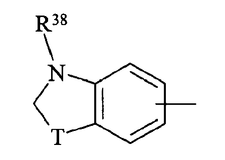 et R³⁸, T, X, Y, R¹, R² et Het représentent des groupes variables.

L'invention concerne également leurs méthodes de préparation, les préparations pharmaceutiques les contenant et leur utilisation à des fins thérapeutiques, en particulier en tant qu'inhibiteurs de calpaïnes et/ou piégeurs de formes réactives de l'oxygène, de manière sélective ou non.

## Description

La présente invention concerne de nouveaux dérivés hétérocycliques présentant une activité inhibitrice des calpaïnes et/ou une activité piégeuse des formes réactives de l'oxygène (ROS pour "reactive oxygen species "). L'invention concerne également leurs méthodes de préparation, les préparations pharmaceutiques les contenant et leur utilisation à des fins thérapeutiques, en particulier en tant qu'inhibiteurs de calpaïnes et piégeurs de formes réactives de l'oxygène de manière sélective ou non.

Compte tenu du rôle potentiel des calpaïnes et des ROS en physiopathologie, les nouveaux dérivés selon l'invention peuvent produire des effets bénéfiques ou favorables dans le traitement de pathologies où ces enzymes et / ou ces espèces radicalaires sont impliquées, et notamment :
- les maladies inflammatoires et immunologiques comme par exemple l'arthrite rhumatoïde, les pancréatites, la sclérose en plaques, les inflammations du système gastro-intestinal (colite ulcérative ou non, maladie de Crohn),
- les maladies cardio-vasculaires et cérébro-vasculaires comprenant par exemple l'hypertension artérielle, le choc septique, les infarctus cardiaques ou cérébraux d'origine ischémique ou hémorragique, les ischémies ainsi que les troubles liés à l'agrégation plaquettaire,
- les troubles du système nerveux central ou périphérique comme par exemple les maladies neurodégénératives où l'on peut notamment citer les traumatismes cérébraux
ou de la moelle épinière, l'hémorragie sub arachnoïde, l'épilepsie, le vieillissement, les démences séniles, y compris la maladie d'Alzheimer, la chorée de Huntington, la maladie de Parkinson, les neuropathies périphériques,
- l'ostéoporose,
- les dystrophies musculaires,
- les maladies prolifératives comme par exemple l'athérosclérose ou la resténose,
- la cataracte,
- les transplantations d'organes,
- les maladies auto-immunes et virales comme par exemple le lupus, le sida, les infections parasitaires et virales, le diabète et ses complications, la sclérose en plaques,
- le cancer,
- toutes les pathologies caractérisées par une production excessive des ROS et / ou une activation des calpaïnes.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des calpaïnes (Trends Pharmacol. Sci. (1994) 15, 412419 ; Drug News Perspect (1999) 12, 73-82). A titre d'exemple, les lésions cérébrales associées à l'infarctus cérébral ou au traumatisme crânien expérimental sont réduites par des agents antioxydants (Acta. Physiol. Scand. (1994) 152, 349-350 ; J. Cereb. Blood Flow Metabol. (1995) 15, 948-952 ; J Pharmacol Exp Ther (1997) 2, 895-904) ainsi que par des inhibiteurs de calpaïnes (Proc Natl Acad Sci U S A (1996) 93, 3428-33 ; Stroke, (1998) 29, 152-158; Stroke (1994) 25, 2265-2270).

La présente invention a donc pour objet des composés de formule générale (I) 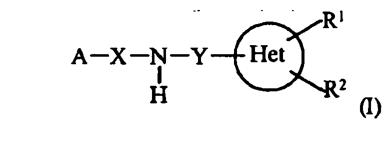 dans laquelle
R¹ représente un atome d'hydrogène, un radical -OR³, -SR³, oxo ou un acétal cyclique,
   dans lequel R³ représente un atome d'hydrogène, un radical alkyle, arylalkyle, hétérocycloalkylcarbonyle, alkylcarbonyle, arylcarbonyle ou aralkylcarbonyle,
   dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵ ;
   R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
R² représente un atome d'hydrogène, un radical alkyle, aryle ou aralkyle, le groupement aryle étant éventuellement substitué par un ou plusieurs radicaux identiques ou différents choisis parmi : -OR⁶, -NR⁷R⁸, halogène, cyano, nitro ou alkyle,
   dans lequel R⁶, R⁷ et R⁸ représentent, indépendamment, un atome d'hydrogène, un radical alkyle, aryle, aralkyle, alkylcarbonyle, arylcarbonyle ou aralkylcarbonyle;
A représente
   un radical A1 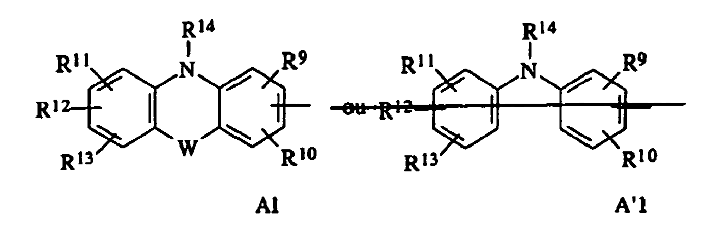 dans lequel R⁹, R¹⁰, R¹¹, R¹², R¹³ représentent, indépendamment, un atome d'hydrogène, un halogène, le groupe OH, un radical alkyle, alkoxy, cyano, nitro ou -NR¹⁵R¹⁶,
   R¹⁵ et R¹⁶ représentent, indépendamment, un atome d'hydrogène, un radical alkyle ou un groupe -COR¹⁷, ou bien R¹⁵ et R¹⁶ forment ensemble avec l'atome d'azote auquel ils sont rattachés un hétérocycle éventuellement substitué,
   R¹⁷ représente un atome d'hydrogène, un radical alkyle, alkoxy ou -NR¹⁸R¹⁹,
   R¹⁸ et R¹⁹ représentent, indépendamment, un atome d'hydrogène ou un radical alkyle, ou bien R¹⁸ et R¹⁹ forment ensemble avec l'atome d'azote auquel ils sont rattachés un hétérocycle éventuellement substitué,
   R¹⁴ représente un atome d'hydrogène, un radical alkyle ou un groupe -COR²⁰,
   R²⁰ représente un atome d'hydrogène, un radical alkyle, alkoxy, aryle, aralkyle, hétérocycloalkyle ou -NR²¹R²²,
   dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵;
R²¹ et R²² représentent, indépendamment, un atome d'hydrogène ou un radical alkyle, ou bien R²¹ et R²² forment ensemble avec l'atome d'azote auquel ils sont rattachés un hétérocycle éventuellement substitué,
W représente une liaison, O ou S ou encore un radical -NR²³, dans lequel R²³ représente un atome d'hydrogène ou un radical alkyle ;
X représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Y représente -(CH₂)ₚ- , -C(R⁵³R⁵⁴)-(CH₂)ₚ-, -C(R⁵³R⁵⁴)-CO- ;
R⁵³ et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Het représente le radical tétrahydrofuran-3-yl.
   ainsi que les sels d'addition avec les acides minéraux et organiques ou avec les bases minérales et organiques desdits composés de formule générale (I),
à l'exclusion des composés de formule (I) dans laquelle lorsque Het représente tétrahydrofuranne ou tétrahydropyranne, R¹ le radical OR³ avec R³ représentant un atome d'hydrogène, un radical alkyle, arylalkyle, hétérocycloalkylcarbonyle dont le radical hétérocycloalkyl est branché par un atome de carbone, alkylcarbonyle, arylcarbonyle ou aralkylcarbonyle, R² un hydrogène et Y le radical -(CH₂)ₚ- avec p = 0, alors X représente -CO-N(R⁴⁵)-C(R⁴⁶R⁴⁷)-CO- avec R⁴⁵=R⁴⁶=H.

Dans certains cas, les composés selon la présente invention peuvent comporter des atomes de carbone asymétriques (de configuration "R" ou "S"). Par conséquent, la présente invention inclut les formes énantiomères, diastéréoisomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères (ou diastéréoisomères) et leurs mélanges sont représentés.

Par alkyle, lorsqu'il n'est pas donné plus de précision, on entend un radical alkyle linéaire ou ramifié comptant de 1 à 6 atomes de carbone comme, par exemple, les radicaux méthyle, éthyle, propyle, isopropyle, butyle, isobutyle, sec-butyle et tert-butyle, pentyle, néopentyle, isopentyle, hexyle, isohexyle. Les radicaux alkoxy peuvent correspondre aux radicaux alkyle indiqués ci-dessus comme par exemple les radicaux méthoxy, éthoxy, propyloxy ou isopropyloxy mais également butoxy linéaire, secondaire ou tertiaire. De même les radicaux alkylthio peuvent correspondre aux radicaux alkyle indiqués ci-dessus comme par exemple méthylthio ou éthylthio.

Par alkényle, lorsqu'il n'est pas donné plus de précision, on entend un radical alkyle linéaire ou ramifié comptant de 1 à 6 atomes de carbone et présentant au moins une insaturation (double liaison). Par halogène, on entend les atomes de fluor, de chlore, de brome ou d'iode.

Par aryle, on entend un système carbocyclique ou hétérocyclique comprenant au moins un cycle aromatique, un système étant dit hétérocyclique lorsque l'un au moins des cycles qui le composent comporte un hétéroatome (O, N ou S). Comme exemple de radical aryle carbocyclique, on peut citer phényle ou naphtyle. Comme exemple de radical aryle hétérocyclique (ou hétéroaryle), on peut citer thiényle, furyle, pyrrolyle, imidazolyle, pyrazolyle, isothiazolyle, thiazolyle, isoxazolyle, oxazolyle, pyridyle, pyrazinyle, pyrimidyle, benzothiényle, benzofuryle et indolyle.

Le terme hétérocycle (ou hétérocycloalkyle), représenté par exemple par les radicaux Het ou Z, représente de préférence un hétérocycle mono ou bicyclique, saturé ou insaturé, comportant de 1 à 5 hétéroatomes choisis parmi O, S, N. L'atome d'azote peut éventuellement être substitué par un radical choisi parmi : alkyle, aryle, aralkyle et alkylcarbonyle. Comme exemple d'hétérocycle saturé, on peut citer : tétrahydrofuranne, tétrahydropyranne, oxétane, oxépane, tétrahydrothiophène, tétrahydrothiopyranne, thiétane, pyrrolidine, pipéridine, azétidine, 1,3-dioxanne, 1,3-dioxolanne, 1,3-dithiolanne, 1,3-dithianne, 1,3-oxathiolannc, 1,3-oxazolidine, 1,3-imidazolidine ou 1,3-thiazolidine. Comme exemple d'hétérocycle insaturé, on peut citer : thiophène, furane, pyrrole, imidazole, pyrazole, isothiazole, thiazole, isoxawle, oxazole, pyridine, pyrazine, pyrimidine, benzimidazole, benzofuranne, benzopyranne, 1,3-benzothiazole, benzoxazole, quinoléine.

Les radicaux arylalkyles (ou aralkyles) désignent les radicaux dans lesquels respectivement les radicaux aryle et alkyle sont tels que définis ci-dessus comme par exemple benzyle, phenéthyle ou naphtylméthyle. Les radicaux aralkoxy (aryl-alkoxy) désignent les radicaux dans lesquels respectivement les radicaux aryle et alkoxy sont tels que définis ci-dessus comme par exemple benzyloxy ou phényléthoxy. Les radicaux arylalkylthio désignent les radicaux dans lesquels respectivement les radicaux aryle et alkylthio sont tels que définis ci-dessus comme par exemple benzylthio.

Les radicaux alkylcarbonyle, hétérocycloalkylcarbonyle, arylcarbonyle ou aralkylcarbonyle désignent les radicaux dans lesquels respectivement les radicaux alkyle, hétérocycloalkyle, aryle et aralkyle ont la signification indiquée précédemment.

Dans le cas de radicaux de formule -NRⁱR^{j} où Rⁱ et R^{j} forment ensemble avec l'atome d'azote auquel ils sont rattachés un hétérocycle éventuellement substitué, l'hétérocycle est de préférence saturé et comprend de 4 à 7 chaînons et de 1 à 3 hétéroatomes incluant l'atome d'azote déjà présent, les hétéroatomes supplémentaires étant choisis indépendamment dans le groupe constitué des atomes O, N et S. Ledit hétérocycle peut être, par exemple, le cycle azétidine, pyrrolidine, pipéridine, pipérazine, morpholine ou thiomorpholine. Ledit hétérocycle peut être substitué par un ou plusieurs substituants identiques ou différents choisis parmi le groupe hydroxy, un radical alkyle, aryle, aralkyle ou alkoxy ou un atome d'halogène.

L'invention a plus particulièrement pour objet des composés de formule (I) telle que définie ci-dessus, dans laquelle représente l'atome d'hydrogène, le radical -OR³ ou oxo.

L'invention a plus particulièrement pour objet également des composés de formule (I) telle que définie ci-dessus, dans laquelle X représente -(CH₂)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO- ou -Z-CO-,
et préférentiellement lorsque R⁴⁵ et R⁴⁷ représentent l'atome d'hydrogène, R⁴⁶ l'atome d'hydrogène, un radical alkyle ou phényle, D le radical phénylène et Z le radical thiazole.

L'invention a plus particulièrement pour objet également des composés de formule (I) telle que définie ci-dessus, dans laquelle R² représente un atome d'hydrogène ou un radical aralkyle, et de préférence le radical benzyle.

L'invention a plus particulièrement pour objet également des composés de formule (I) telle que définie ci-dessus, dans laquelle A représente A1 avec W représentant l'atome de soufre, et de manière préférentielle le radical de formule

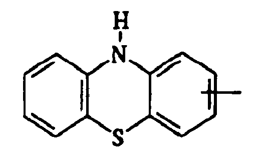

Plus particulièrement également, l'invention a pour objet des composés décrits ci-après dans les exemples et préférentiellement les produits répondant aux formules suivantes :
N-[(1S)-1-({[(3S)-2-hydroxytétrahydro-3-furanyl]amino}carbonyl)-3-méthylbutyl]-10*H-*phénothiazine-2-carboxamide ;
Acétate de (3*S*)-3-({(2*S*)-4-méthyl-2-[(10*H*-phénothiazin-2-ylcarbonyl)-amino]pentanoyl} amino)tétrahydro-2-furanyl ;
N-[(3S)-2-hydroxytétrahydro-3-furanyl]-2-(10*H*-phénothiazin-2-yl)-1,3-thiazole-4-carboxamide ;
N-[4-({[(3S)-2-hydroxytétrahydro-3-furanyl]amino}carbonyl)phényl]-10*H-*phénothiazine-2-carboxamide ;
N-[(1S)-1-({[(3S)-2-hydroxytétrahydro-3-furanyl]amino}carbonyl)-3-méthylbutyl]-10*H*-phénothiazine-1-carboxamide ;
Pivalate de (3S)-3-({(2S)-4-méthyl-2-[(10*H*-phénothiazin-2-ylcarbonyl)amino] pentanoyl}amino)tétrahydro-2-furanyl ;
3,3-diméthylbutanoate de (3S)-3-({(2S)-4-méthyl-2-[(10*H*-phénothiazin-2-ylcarbonyl) amino]pentanoyl)amino)tétrahydro-2-furanyl ;
(3S)-3-({(2S)-4-méthyl-2-[(10*H*-phénothiazin-2-ylcarbonyl)amino]pentanoyl}amino) tétrahydro-2-furanyl benzoate ;
(3S)-3-({(2S)-4-méthyl-2-[(10*H*-phénothiazin-2-ylcarbonyl)amino]pentanoyl}amino) tétrahydro-2-furanyl phénylacetate ;
(3*S*)-3-({(2*S*)-4-méthyl-2-[(10*H*-phénothiazin-2-ylcarbonyl)amino]pentanoyl}aminn) tétrahydro-2-furanyl (2*S*)-2-(diméthylamino)-3-phénylpropanoate :
4-morpholinecarboxylate de (3S)-3-({(2S)-4-méthyl-2-[(10*H*-phénothiazin-2-ylcarbonyl) amino]pentanoyl]amino)tétrahydro-2-furanyl ;

L'invention a plus particulièrement pour objet également les composés répondant à l'une des formules suivantes :
N-[(1S)-1-({[(3S)-2-hydroxytétrahydro-3-furanyl]amino}carbonyl)-3-méthylbutyl]-10H-phénothiazine-2-carboxamide ;
Acétate de (3*S*)-3-({(2*S*)-4-méthyl-2-[(10*H*-phénothiazin-2-ylcarbonyl)-amino] pentanoyl}amino)tétrahydro-2-furanyl ;
Acétate de (2R,3S)-3-({(2S)-4-méthyl-2-[(10H-phénothiazin-2-ylcarbonyl)amino]-pentanoyl}amino)tétrahydro-2-furanyl ;
Acétate de (2S,3S)-3-({(2S)-4-méthyl-2-[(10H-phénothiazin-2-ylcarbonyl) amino]-pentanoyl}amino)tétrahydro-2-furanyl ; ainsi que les sels d'addition avec les acides minéraux et organiques ou avec les bases minérales et organiques desdits composés.

Les composés de formule I selon l'invention peuvent être préparés suivant plusieurs voies de synthèse selon la définition des groupes variables.

Les composés de formule générale (I) dans laquelle Het représente le cycle tétrahydrofuranne et Y le radical -(CH₂)ₚ, peuvent être préparés selon le schéma suivant : 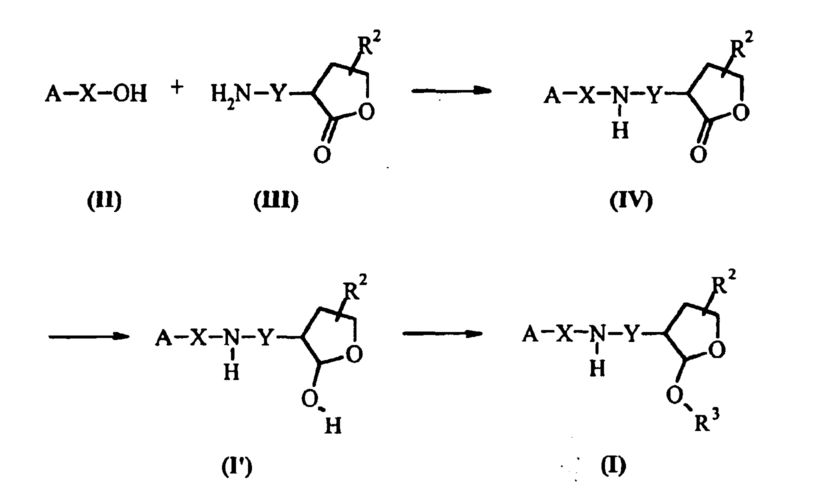 dans lesquels A, X, R² et R³ sont tels que décrits ci-dessus,
par condensation des acides de formule générale (II) sur les aminés de formule générale (III), dans les conditions classiques de la synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexyicarbodiimide (DCC), le 1,1'-carbonyldiimidazole (CDI) (J. Med. Chem. (1992), 35 (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)) pour conduire aux carboxamides intermédiaires de formule générale (IV). Le cycle lactonique des intermédiaires de formule générale (IV) est ensuite réduit à l'aide d'un agent réducteur tel que, par exemple, l'hydrure de Diisobutylaluminium (DIBAL), dans un solvant inerte tel que, par exemple, THF ou CH₂Cl₂, à une température variant de 0 à -78° C. Le dérivé lactol de formule générale (I') ainsi obtenu peut être acylé à l'aide, par exemple, d'un chlorure d'acide (R³-Cl) ou d'un anhydride d'acide (anhydride acétique, chlorure de benzoyle, ...) en présence d'une base telle que, par exemple, la triéthylamine, dans un solvant inerte comme par exemple CH₂Cl₂ pour conduire au composé de formule générale (I).

Les composés de formule générale (I) dans laquelle Het représente le cycle tétrahydrofuranne, X le radical -(CH₂)ₙ- (n = 0) et Y le radical -(CH₂)ₚ- (p = 0), peuvent également être préparés selon le schéma suivant : 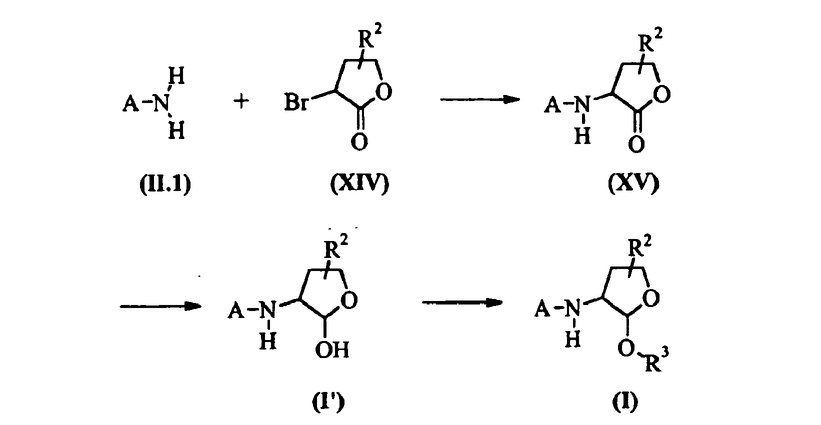 dans lesquels A, R² et R³ sont tels que décrits ci-dessus,
par substitution nucléophile de l'halogène des lactones de formule générale (XV) à l'aide des amines de formule générale (II.1), en chauffant le mélange réactionnel à une température variant de 50 à 110°C dans un solvant inerte tel que par exemple, l'acétonitrile ou le DMF, pendant une durée variant de 30 minutes à 5 heures, pour conduire aux intermédiaires de formule générale (XV). La réduction de la fonction lactone suivie de l'acylation du lactol de formule générale (I') sont effectuées dans les conditions précédemment décrites.

Les composés de formule générale (I) dans laquelle Het représente le cycle tétrahydrofuranne, X le radical -N(R⁴⁵)-(CH₂)ₙ-CO- (n = 0) et Y le radical -(CH₂)ₚ- (p = 0) sont des urées qui peuvent être préparées selon le schéma synthétique suivant : 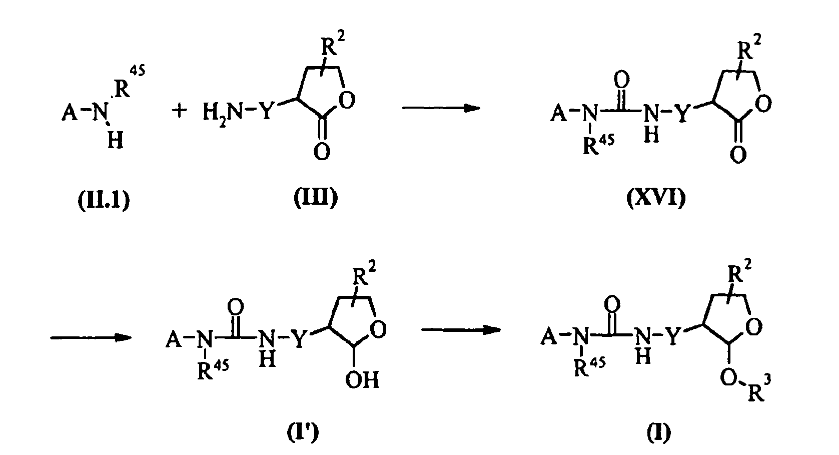 dans lesquels A, R² et R³ sont tels que décrits ci-dessus,
par condensation des amines de formule générale (II.1) avec les amines de formule générale (III) en présence de triphosgène et d'une base telle que, par exemple, la diisopropyléthylamine dans un solvant inerte tel que le dichlorométhane selon un protocole expérimental décrit dans J. Org. Chem. (1994) 59 (7), 1937-1938. Le cycle lactonique des urées de formule générale (XVI) est ensuite réduit et modifié dans les conditions expérimentales précédemment décrites pour conduire aux composés de formule générale (I).

Les composés de la présente invention possèdent d'intéressantes propriétés pharmacologiques : ils présentent une activité inhibitrice des calpaïnes et / ou une activité piégeuse des formes réactives de l'oxygène.

Les composés de la présente invention peuvent ainsi être utilisés dans différentes applications thérapeutiques. Ils peuvent produire des effets bénéfiques ou favorables dans le traitement de pathologies où ces enzymes et/ou ces espèces radicalaires sont impliquées.

Ces propriétés rendent les produits de formule 1 aptes à une utilisation pharmaceutique. La présente demande a également pour objet, à titre de médicaments, les produits de formule 1 telle que définie ci-dessus, ainsi que les sels d'addition avec les acides minéraux ou organiques ou les bases minérales et organiques pharmaceutiquement acceptables desdits produits de formule I, ainsi que les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Certains composés de la formule générale 1 précédemment décrite, sont couverts par la demande EP 641800. Les composés de cette demande présentent une activité inhibitrice de cathepsine L qui est différente de l'activité inhibitrice des calpaïnes et / ou de l'activité piégeuse des formes réactives de l'oxygène.

L'invention a donc également pour objet l'utilisation de composés de formule (Iₐ) telle que définie ci-dessus, 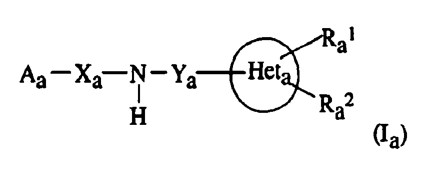 sous forme racémique, d'énantiomères, de diastéréoisomères ou toutes combinaisons de ces formes, dans laquelle
Rₐ¹ représente un atome d'hydrogène, un radical -OR³, -SR³, oxo ou un acétal cyclique,
   dans lequel R³ représente un atome d'hydrogène, un radical alkyle, arylalkyle, hétérocycloalkylcarbonyle, alkylcarbonyle, arylcarbonyle ou aralkylcarbonyle,
   dans lesquels les radicaux alkyle, aryle ou hétérocycloalkyle sont éventuellement substitués par un ou plusieurs substituants identiques ou différents choisis parmi : alkyle, OH, aUcoxy, nitro, cyano, halogène ou -NR⁴R⁵;
   R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
Rₐ² représente un atome d'hydrogène, un radical alkyle, aryle ou aralkyle, le groupement aryle étant éventuellement substitué par un ou plusieurs radicaux identiques ou différents choisis parmi : -OR⁶, -NR⁷R⁸, halogène, cyano, nitro ou alkyle,
   dans lequel R⁶, R⁷ et R⁸ représentent, indépendamment, un atome d'hydrogène, un radical alkyle, aryle, aralkyle, alkylcarbonyle, arylcarbonyle ou aralkylcarbonyle;
Aₐ représente un radical A1 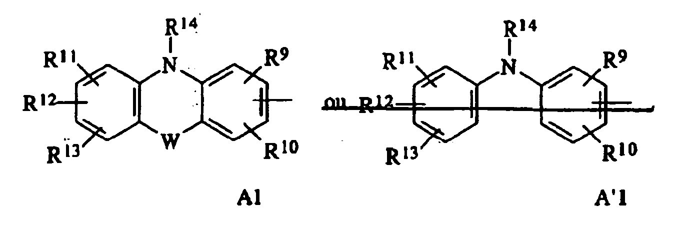 dans lequel R⁹, R¹⁰, R¹¹, R¹², R¹³ représentent, indépendamment, un atome d'hydrogène, un halogène, le groupe OH, un radical alkyle, alkoxy, cyano, nitro ou -NR¹⁵R¹⁶,
   R¹⁵ et R¹⁶ représentent, indépendamment, un atome d'hydrogène, un radical alkyle ou un groupe -COR¹⁷, ou bien R¹⁵ et R¹⁶ forment ensemble avec l'atome d'azote auquel ils sont rattachés un hétérocycle éventuellement substitué,
   R¹⁷ représente un atome d'hydrogène, un radical alkyle, alkoxy ou -NR¹⁸R¹⁹,
   R¹⁸ et R¹⁹ représentent, indépendamment, un atome d'hydrogène ou un radical alkyle, ou bien R¹⁸ et R¹⁹ forment ensemble avec l'atome d'azote auquel ils sont rattachés un hétérocycle éventuellement substitué,
   R¹⁴ représente un atome d'hydrogène, un radical alkyle ou un groupe -COR²⁰,
   R²⁰ représente un atome d'hydrogène, un radical alkyle, alkoxy, aryle, aralkyle, hétérocycloalkyle ou -NR²¹R²²,
   dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵;
   R²¹ et R²² représentent, indépendamment, un atome d'hydrogène ou un radical alkyle, ou bien R²¹ et R²² forment ensemble avec l'atome d'azote auquel ils sont rattachés un hétérocycle éventuellement substitué,
   W représente une liaison, O ou S ou encore un radical -NR²³, dans lequel R²³ représente un atome d'hydrogène ou un radical alkyle ;
Xₐ représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁹ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Yₐ représente -(CH₂)ₚ-, -C(R⁵³R⁵⁴)-(CH₂)ₚ-, -C(R⁵³R⁵⁴)-CO- ;
R⁵³ et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Hetₐ représente le radical tétrahydrofuran-3-yl. ainsi que des sels d'addition avec les acides minéraux et organiques ou avec les bases minérales et organiques desdits composés de formule générale (I),
   pour la préparation de médicaments pour le traitement de pathologies où les calpaïnes et / ou les formes réactives de l'oxygène sont impliquées.

L'invention a plus particulièrement pour objet l'utilisation de composés de formule (Iₐ) telle que définie ci-dessus, pour la préparation de médicaments pour le traitement de pathologies où les formes réactives de l'oxygène sont impliquées. L'invention a plus particulièrement pour objet également l'utilisation de composés de formule (Iₐ) telle que définie ci-dessus, pour la préparation de médicaments pour le traitement de pathologies où les formes réactives de l'oxygène et les calpaïnes sont impliquées. L'invention concerne donc l'utilisation de composés de formule (Iₐ) telle que définie ci-dessus, pour la préparation de médicaments pour le traitement de pathologies comme les maladies inflammatoires et immunologiques, les maladies cardio-vasculaires et cérébro-vasculaires, les troubles du système nerveux central ou phériphérique, l'ostéoporose, les dystrophies musculaires, les maladies prolifératives, la cataracte, les transplantations d'organes, les maladies auto-immunes et virales, le cancer, et toutes les pathologies caractérisées par une production excessive des ROS et / ou une activation des calpaïnes.

L'invention a plus particulièrement pour objet l'utilisation de composés de formule (Iₐ) telle que définie ci-dessus, caractérisée en ce que R¹ représente l'atome d'hydrogène, le radical -OR³ ou oxo.

L'invention a plus particulièrement pour objet l'utilisation de composés de formule (Iₐ) telle que définie ci-dessus, caractérisée en ce que X représente -(CH₂)ₙ-, -(CH₂)ₙ-CO-, -O-(CH₂)ₙ-CO-, -CO-N(R⁴⁵)-D-CO-, -Z-CO-, -N(R⁴⁵)-CO-(CH₂)ₙ-CO-, -N(R⁴⁵)-CO-C(R⁴⁶R⁴⁷)-CO-, -N(R⁴⁵)-(CH₂)ₙ-CO-, -N(R⁴⁵)-CO-NH-C(R⁴⁶R⁴⁷)-CO- ou -CO-N(R⁴⁵)-C(R⁴⁶R⁴⁷)-CO et préférentiellement lorsque R⁴⁵ et R⁴⁷ représentent l'atome d'hydrogène, R⁴⁶ l'atome d'hydrogène, un radical alkyle ou phényle, D le radical phénylène et Z le radical thiazole.

L'invention a plus particulièrement pour objet l'utilisation de composés de formule (Iₐ) telle que définie ci-dessus caractérisée en ce que R² représente un atome d'hydrogène ou un radical aralkyle, et de préférence le radical benzyle.

L'invention a plus particulièrement pour objet l'utilisation de composés de formule (Iₐ) telle que définie ci-dessus caractérisée en ce que A représente A1 avec W représentant l'atome de soufre, manière préférentielle A représente le radical

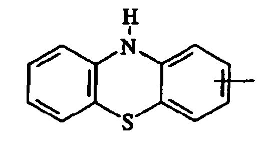

Plus particulièrement également, l'invention a pour objet l'utilisation telle que définie ci-dessus, de composés de formule (Iₐ) tels que décrits dans les exemples et préférentiellement les composés qui répondent à l'une des formules suivantes :
N-[(1S)-1-({[(3S)-2-hydroxytétrahydro-3-furanyl]amino}carbonyl)-3-méthylbutyl]-10*H-*phénothiazine-2-carboxamide ;
Acétate de (3*S*)-3-({(2*S*)-4-méthyl-2-[(10*H*-phénothiazin-2-ylcarbonyl)-amino]pentanoyl}amino)tétrahydro-2-furanyl ;
N-[(3S)-2-hydroxytétrahydro-3-furanyl]-2-(10*H*-phénothiazin-2-yl)-1,3-thiazole-4-carboxamide ;
N-[4-({[(3S)-2-hydroxytétrahydro-3-furanyl]amino}carbonyl)phényl]-10*H-*phénothiazine-2-carboxamide ;
N-[(1S)-1-({[(3S)-2-hydroxytétrahydro-3-furanyl]amino}carbonyl)-3-méthylbutyl]-10*H*-phénothiazine-1-carboxamide ;
Pivalate de (3S)-3-({(2S)-4-méthyl-2-[(10*H*-phénothiazin-2-ylcarbonyl)amino] pentanoyl]}amino)tétrahydro-2-furanyl ;
3,3-diméthylbutanoate de (3S)-3-({(2S)-4-méthyl-2-[(10*H*-phénothiazin-2-ylcarbonyl) amino]pentanoyl}amino)tétrahydro-2-furanyl ;
(3S)-3-({(2S)-4-méthyl-2-[(10*H*-phénothiazin-2-ylcarbonyl)amino]pentanoyl}amino) tétrahydro-2-furanyl benzoate ;
(3S)-3-({(2S)-4-méthyl-2-[(10*H*-phénothiazin-2-ylcarbonyl)amino]pentanoyl}amino) tétrahydro-2-furanyl phénylacetate ;
(3*S*)-3-({(2*S*)-4-méthyl-2-[(10*H*-phénothiazin-2-ylcarbonyl)amino]pentanoyl}amino) tétrahydro-2-furanyl (2*S*)-2-(diméthylamino)-3-phénylpropanoate ;
4-morpholinecarboxylate de (3S)-3-({(2S)-4-méthyl-2-[(10*H*-phénothiazin-2-ylcarbonyl) amino]pentanoyl}amino)tétrahydro-2-furanyl;

L'invention a plus particulièrement pour objet également l'utilisation de composés de formule (Iₐ) telles que définies ci-dessus et caractérisés en ce qu'ils répondent à l'une des formules suivantes :
N-[(1S)-1-({[(3S)-2-hydroxytétrahydro-3-furanyl]amino}carbonyl)-3-méthylbutyl]-10H-phénothiazine-2-carboxamide ;
Acétate de (3*S*)-3-({(2*S*)-4-méthyl-2-[(10*H*-phénothiazin-2-ylcarbonyl)-amino] pentanoyl}amino)tétrahydro-2-furanyl ;
Acétate de (2R,3S)-3-({(2S)-4-méthyl-2-[(10H-phénothiazin-2-ylcarbonyl)amino]-pentanoyl}amino)tétrahydro-2-furanyl ;
Acétate de (2S,3S)-3-({(2S)-4-méthyl-2-[(10H-phénothiazin-2-ylcarbonyl) amino]-pentanoyl}amino)tétrahydro-2-furanyl.

Les intermédiaires de synthèse non commerciaux de formule (II), (III) et (V) peuvent être préparés selon les différentes voies de synthèse ci-dessous :
1) Synthèse des intermédiaires (II) :
   Les acides carboxyliques de formule générale (II), dans lesquels A, X, D, n, R⁴⁵, R⁴⁶ et R⁴⁷ sont tels que décrits ci-dessus, sont accessibles à partir des schémas synthétiques suivants :
1.1) A partir de A-NH(R⁴⁵) :
   La préparation des acides carboxyliques de formule générale (II) peut être effectuée, dans ce cas, à partir de 3 dérivés acides-esters différents (II.2), (II.4) et (II.6) :

La condensation des anilines de formule générale (II.1) avec les acide-esters (Alk = Alkyle) commerciaux de formule générale (II.2), schéma 1.1, est effectuée par condensation peptidique classique. Le carboxamide intermédiairement obtenu (II.3) est ensuite saponifié pour conduire aux acides carboxyliques de formule générale (II). La synthèse des intermédiaires de formule générale (II.1) est décrite plus loin.

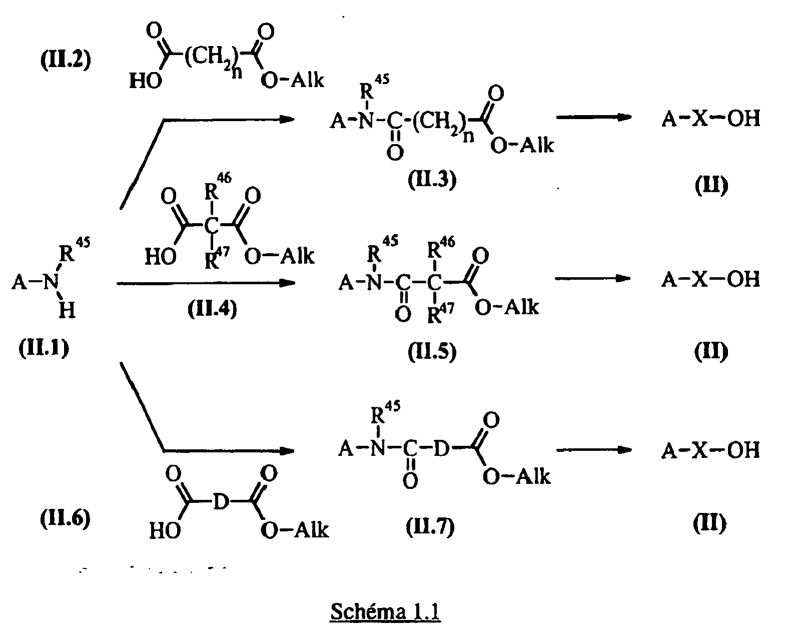

La synthèse des acides carboxyliques de formule générale (II) peut également être effectuée par condensation des anilines de formule générale (II.1) avec les dérivés d'acide-esters de formule générale (II.4) dans les conditions précédemment décrites. Cette condensation est suivie d'une saponification classique pour conduire aux acides de formule générale (II). La synthèse des intermédiaires de formule générale (II.4) est décrite plus loin.

La condensation des amines de formule générale (II.1) avec les acides aromatiques commerciaux de formule générale (II.6), dans les conditions de synthèse peptidique déjà décrites, permet après saponification des intermédiaires de formule générale (II.7) d'obtenir également les acides carboxyliques de formule générale (II).

Alternativement les acides carboxyliques de formule générale (II), sont également accessibles par l'ouverture d'anhydrides cycliques tels que par exemple, l'anhydride succinique, à l'aide des amines de formule générale (II.1) selon un protocole expérimental décrit dans la littérature (J. Amer. Chem. Soc. (1951) 73, 4007).

### 1.1.1) Préparation des intermédiaires (II.1) :

Les anilines de formule générale (II.1), non commerciales, dérivés d'indoline ou de 1,2,3,4-tétrahydroquinoline, schéma 1.1.1, dans lesquelles T et R³⁸ sont tels que définis ci-dessus, peuvent être préparées à partir des dérivés nitro correspondants de formule générale (II.1.1). La 6-nitro-1,2,3,4-tétrahydroquinoline est décrite dans Can. J. Chem. (1952), 30, 720-722. L'alkylation de l'amine est classiquement effectuée par une base forte telle que, par exemple, NaH, dans un solvant aprotique polaire tel que, par exemple, le DMF en présence d'un dérivé halogéné R³⁸-Hal, tels que par exemple, le chlorure de 3-diméthylaminopropane ou le bromure de benzyle. Le dérivé nitro de formule générale (II.1.2) intermédiairement obtenu est ensuite réduit, par exemple, par le Nickel de Raney en présence d'hydrate d'hydrazine pour conduire aux anilines de formule générale (II.1).

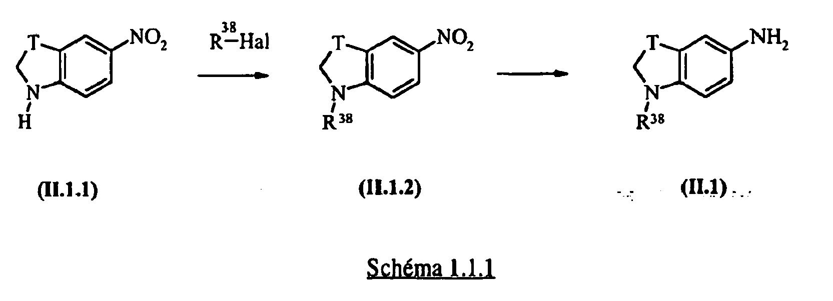

Par ailleurs, certains dérivés des phénylènediamines de formule générale (II.1), non commerciaux, peuvent être préparés selon Farmaco (1951) 6, 713-717.

Dans le cas particulier où A est un dérivé phénolique (A=A2), les anilines de formule générale (II.1) sont obtenues par hydrogénation, en présence de Pd/C, des dérivés nitrophénols précurseurs. Les dérivés nitrés des di-alkyl phénols sont accessibles selon les méthodes décrites dans J. Org. Chem. (1968) 33 (1), 223-226 ou J. Med. Chem. (1998), 41,1846-1854.

Les intermédiaires de formule générale (II.1) dans lesquels A'1 est une diphénylamine, sont accessibles à partir des méthodes décrites dans la littérature (Synthesis (1990) 430 ; Indian J. Chem. (1981) 20B, 611-613 ; J. Med. Chem. (1975) 18 (4), 386-391) qui passent par la réduction d'un intermédiaire nitrodiphénylamine. La réduction de la fonction nitro est effectuée classiquement par hydrogénation en présence d'une quantité catalytique de Pd/C pour accéder aux aminodiphénylamines de formule générale (II.1).

Lorsque A est un dérivé carbazole (W représente alors une liaison directe), les méthodes de préparation des aminocarbazoles de formule générale (II.1) passent par la synthèse d'un intermédiaire nitrocarbazole. Ces méthodes sont décrites dans Pharmazie (1993) 48 (11), 817-820 ; Synth. Commun. (1994) 24(1), 1-10 ; J. Org. Chem. (1980) 45, 1493-1496 ; J. Org. Chem. (1964) 29 (8), 2474-2476 ; Org. Prep. Proced. Int. (1981) 13 (6), 419-421 ou J. Org. Chem. (1963) 28, 884. La réduction de la fonction nitro des intermédiaires nitrocarbazoles est, dans ce cas, effectuée de préférence à l'aide d'hydrate d'hydrazine en présence de Nickel de Raney.

Les intermédiaires de formule générale (II.1) dans lesquels A est un dérivé phénothiazine (W représente un atome de soufre), sont accessibles à partir de méthodes de la littérature qui passent par la synthèse d'un dérivé nitrophénothiazine. En particulier la 3-nitrophénothiazine est décrite dans J. Org. Chem. (1972) 37, 2691. La réduction de la fonction nitro pour accéder aux aminophénothiazines de formule générale (II.1) est effectuée classiquement par hydrogénation en présence d'une quantité catalytique de Pd/C dans un solvant tel que l'éthanol.

### 1.1.2) Préparation des intermédiaires (II.4) :

Les acide-esters de formule générale (II.4), schéma 1.1.2, peuvent être préparés à partir des diesters commerciaux de formule générale (II.4.1) selon une méthode décrite dans la littérature (Tetrahedron Asymmetry (1997) 8 (11), 1821-1823).

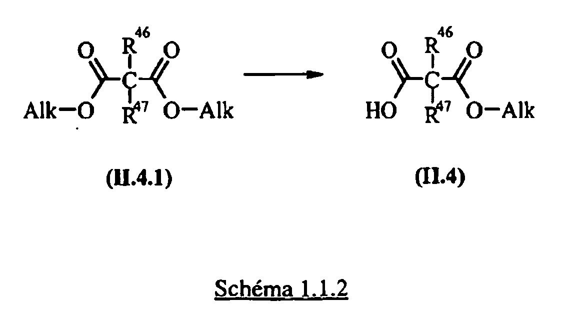

### 1.2) A partir de A-CO₂H :

Les intermédiaires acides carboxyliques de formule générale (II) sont également accessibles à partir de la condensation des acides carboxyliques de formule générale (II.8) avec les amino-esters commerciaux de formule générale (II.9A) ou (II.9B), schéma 1.2, au cours d'une étape de synthèse peptidique précédemment décrite. Les carboxamides intermédiairement obtenus (II.10A) et (II.10B) sont ensuite saponifiés pour conduire aux acides carboxyliques de formule générale (II).

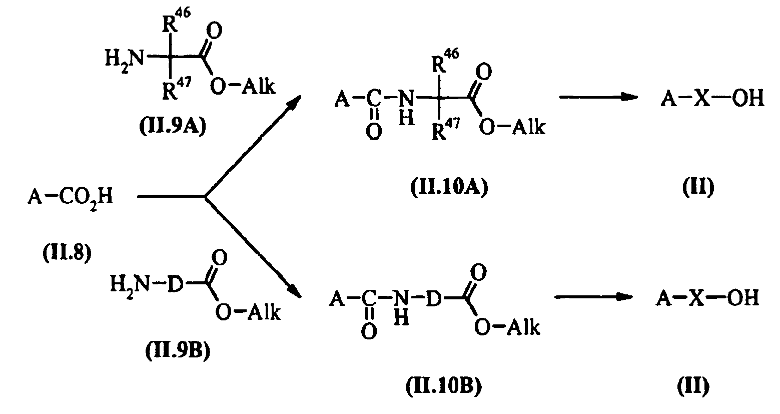

### 1.2.1) Préparation des intermédiaires (II.8) :

Les dérivés carboxyliques de formule générale (II.8), non accessibles commercialement, peuvent être préparés à partir de la littérature (p. ex. : J. Org. Chem. (1961) 26, 1221-1223 ; Acta Chem. Scandinavica (1973) 27, 888-890 ; Can. J. Chem. (1972) 50, 1276-1282 ; J. Med. Chem. (1992) 35(4), 716-724 ; J. Org. Chem. (1989) 54, 560-569 ; J. Med. Chem. (1998) 41(2), 148-156 ; Bull. Soc. Chim. Fr. (1960), 1049-1066)).

### 1.3) A partir de A-OH ou A-SH :

Les acides de formule générale (II) (schéma 1.3) dans lesquels X représente -O-(CH₂)ₙ₋CO-, sont préparés à partir des hydroquinones de formule générale (II.11) obtenues selon la littérature (J. Chem. Soc. Perkin 1 (1981) 303-306). La condensation sur des halogènoesters commerciaux de formule générale (II.12) est effectuée en présence d'une base telle que, par exemple K₂CO₃, en chauffant dans un solvant polaire comme, par exemple, le THF pendant au moins 5 heures. Les esters de formule générale (II.13) intermédiairement obtenus sont ensuite déprotégés (en milieu acide dans le cas des esters de tert-butyle) pour conduire aux acides de formule générale (II).

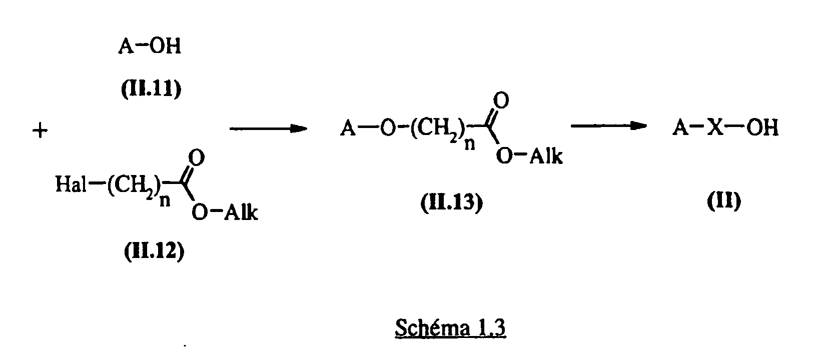

Les acides de formule générale (II) dans lesquels X représente -S-(CH₂)ₙ-CO-, sont préparés selon une méthode de la littérature (J. Med. Chem. (1997) 40 (12), 1906-1918).

### 1.4) A partir de A-CO₂H, lorsque Z représente un hétérocycle avec V = S ou O :

1.4.a) Dans les cas où Z représente un hétérocyle insaturé, les acides carboxyliques de formule générale (II), schéma 1.4a, peuvent être préparés à partir des acides carboxyliques de formule générale (II.8).

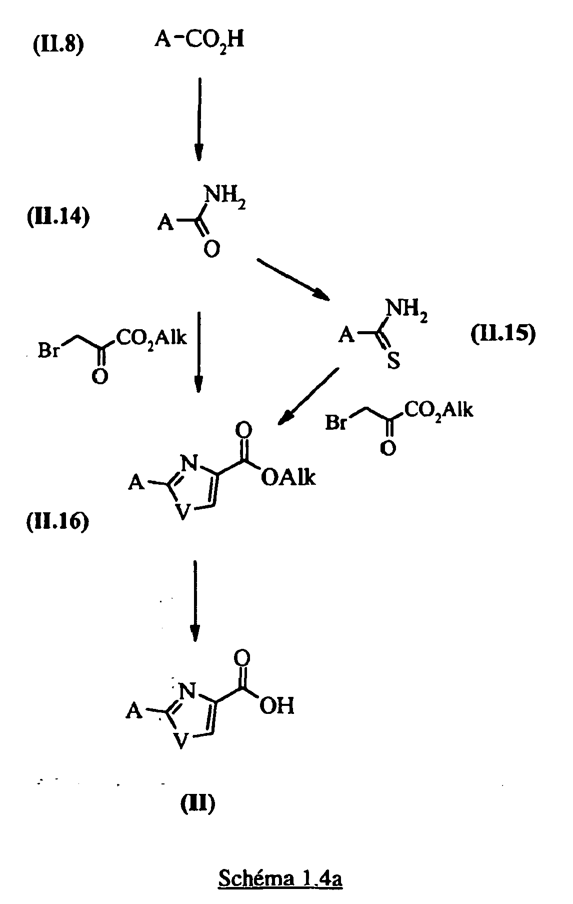

La formation du carboxamide I^{aire} de formule générale (II.14) est effectuée selon un protocole expérimental décrit dans la littérature (Synthesis (1989), 1, 37). Par chauffage, entre 50° C et le reflux du solvant, pendant un temps compris entre une et 15 heures, de l'intermédiaire (II.14) en présence d'un bromopyruvate d'alkyle, on obtient les oxazoles (V = O) de formule générale (II.16). Alternativement, les thiazoles (V = S) de formule générale (II.16), sont accessibles en deux étapes à partir des carboxamides de formule générale (II.14). Ceux-ci en présence du réactif de Lawesson dans un solvant tel que, par exemple, le 1,4-dioxanne, conduisent classiquement aux thiocarboxamides de formule générale (II.15). L'étape de cyclisation est ensuite effectuée en présence de bromopyruvate d'alkyle comme précédemment décrit. Les acides carboxyliques de formule générale (II) sont finalement obtenus par déprotection de la fonction acide dans des conditions classiques.

1.4.b) Dans les cas où Z représente un hétérocycle saturé, et en particulier une thiazolidine, les acides carboxyliques de formule générale (II), schéma 1.4b, sont également accessibles à partir des acides carboxyliques de formule générale (II.8).

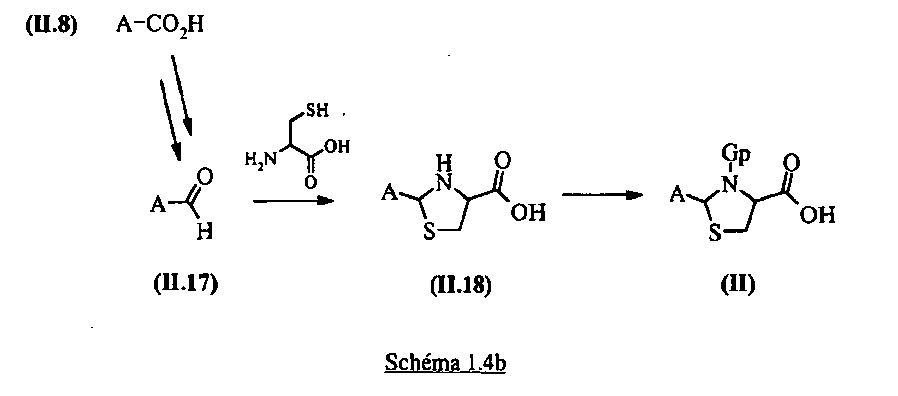

La préparation des aldéhydes de formule générale (II.17) est classiquement effectuée après activation de la fonction acide des intermédiaires de formule générale (II.8) sous forme d'ester ou d'alkylhydroxamate, en présence de DIBAL ou de LiAlH₄ , selon des protocoles expérimentaux de la littérature (p. ex. J. Med. Chem. (1990) 33, 11-13). La réaction de ces aldéhydes avec la cystéine en présence de sels d'acétate conduit directement aux thiazolidines de formule générale (II.18) selon un protocole expérimental décrit dans J. Org. Chem. (1957) 22, 943-946. L'amine du cycle thiazolidine est ensuite protégée sous forme de carbamate (p.ex. Boc) dans les conditions classiques de la littérature pour conduire aux acides carboxyliques de formule générale (II).

### 1.5) A partir de A-N(R⁴⁵)-CO- :

Les acides carboxyliques de formule générale (II), dans lesquels X = -N(R⁴⁵)-(CH₂)ₙ-CO-avec n = 0, sont constitués d'une chaîne fonctionnalisée par une urée, schéma 1.5.

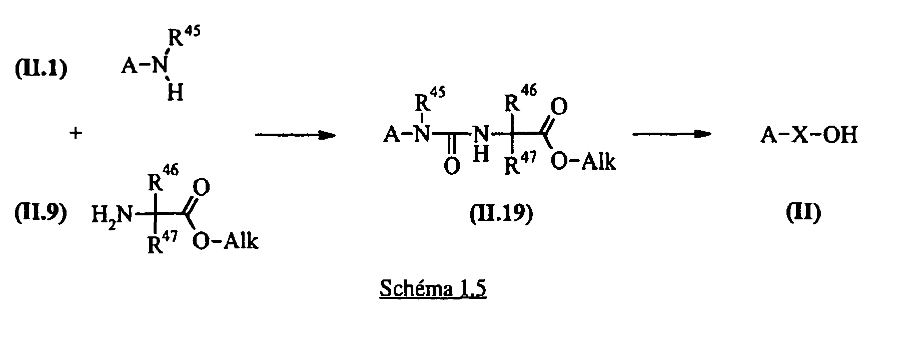

La synthèse de ces urées se fait par condensation des amines de formule générale (II.1) avec les aminoesters de formule générale (II.9) en présence de triphosgène et d'une amine tertiaire selon un protocole expérimental décrit dans la littérature (J. Org. Chem. (1994), 59(7), 1937-1938) pour conduire aux intermédiaires de formule générale (II.19). L'acide carboxylique de formule générale (II) est ensuite classiquement obtenu par déprotection de l'ester intermédiaire.

### 2) Synthèse des intermédiaires (III) :

La préparation des intermédiaires de formule générale (III), schéma 1.4, dans lesquels R² est tel que défini ci-dessus et Y = -(CH₂)ₚ-, avec p = 0, est effectuée à partir des dérivés de l'acide N-Cbz aspartique de formule générale (III.1) dont l'accès est décrit dans la littérature (J. Med. Chem. (1973) 16 (11), 1277-1280). Par chauffage de ces intermédiaires en présence de trioxanne et d'une quantité catalytique d'APTS au reflux d'un solvant tel que, par exemple, le toluène, (Synthesis (1989) 7, 542-544) on obtient les dérivés d'oxazolidinone de formule générale (III.2). La réduction de la fonction acide est alors effectuée à l'aide de B₂H₆.THF dans le THF telle que décrite dans Chem. Pharm. Bull. (1995) 43 (10), 1683-1691 et conduit aux alcools de formule générale (III.3). Ceux-ci sont ensuite traités en milieu basique, et l'intermédiaire (III.4) ainsi généré est cyclisé à l'aide d'un agent de déshydratation classique tel que, par exemple, le Dicyclohexylcarbodiimide pour obtenir la lactone substituée de formule générale (III.5). L'intermédiaire de formule générale (III) est obtenu après coupure du carbamate de benzyle à l'aide de Pd/C sous atmosphère d'hydrogène.

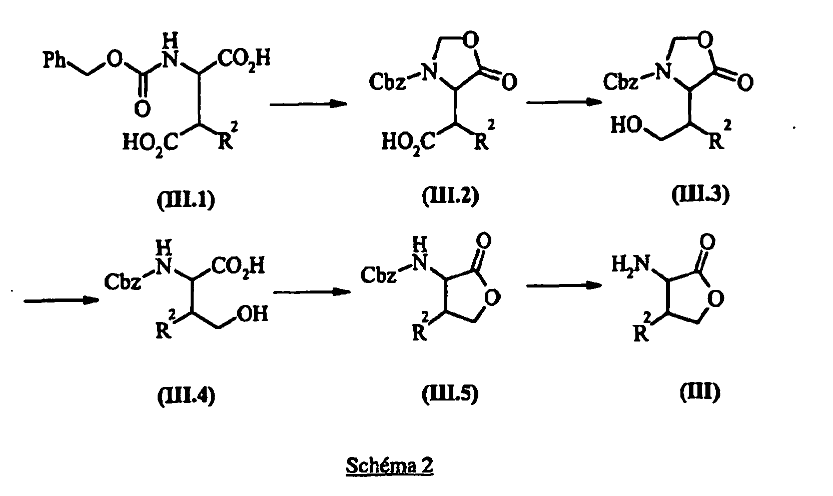

L'invention a également pour objet, à titre de produits industriels nouveaux, et notamment à titre de produits industriels nouveaux destinés à la préparation de produits de formule I, les produits répondant à l'une des formule suivantes :
N-1-(4-anilinophényl)-N-4-[(3S)-2-oxotétrahydro-3-furanyl]succinamide ;
(2S)-2-{[4-(4-anilinoanilino)-4-oxobutanoyl]amino}-4-méthylpentanoate de méthyle ;
N1-(4-anilinophényl)-N4-[(1S)-1-formyl-3-méthylbutyl]succinamide;
benzyl 3-(4-anilinoanilino)-3-oxo-2-phénylpropanoate ;
acide 3-(4-anilinoanilino)-3-oxo-2-phénylpropanoïque ;
N-1-(4-anilinophényl)-N-3-[(3S)-2-oxotétrahydro-3-furanyl]-2-phénylmalonamide ;
3-(4-anilinoanilino)dihydro-2(3H)-furanone ;
(2S)-4-méthyl-2-[(10H-phénothiazin-2-ylcarbonyl)amino]pentanoate de méthyle ;
acide (2S)-4-méthyl-2-[(10H-phénothiazin-2-ylcarbonyl)amino]pentanoïque ;
N-[(1S)-3-méthyl-1-({[(3S)-2-oxotétrahydro-3-furanyl]amino}carbonyl)butyl]-10H-phénothiazine-2-carboxamide ;
2-(10H-phénothiazin-2-yl)-1,3-thiazole-4-carboxylate d'éthyle ;
acide 2-(10H-phénothiazin-2-yl)-1,3-thiazole-4-carboxylique ;
N-[(3S)-2-oxotétrahydro-3-furanyl]-2-(10H-phénothiazin-2-yl)-1,3-thiazole-4-carboxamide ;
4-[(10H-phenothiazin-2-ylcarbonyl)amino]benzoate de méthyle ;
acide 4-[(10H-phénothiazin-2-ylcarbonyl)amino]benzoïque ;
N-[4-({[(3S)-2-oxotétrahydro-3-furanyl]amino}carbonyl)phényl]-10H-phénothiazine-2-carboxamide ;
(2S)-4-méthyl-2-[(10H-phénothiazin-1-ylcarbonyl)amino]pentanoate de méthyle ;
acide (2S)-4-méthyl-2-[(10H-phénothiazin-1-ylcarbonyl)amino]pentanoïque ;
N-[(1S)-1-({[(3S)-2-oxotétrahydro-3-furanyl]amino}carbonyl)-3-méthylbutyl]-10H-phénothiazine-1-carboxamide ;
*N*-[(1*S*)-1-(1,4-dioxa-7-azaspiro[4.4]non-7-ylcarbonyl)-3-méthylbutyl]-10*H-*phénothiazine-2-carboxamide ;
2-(3,5-di-*tert*-butyl-4-hydroxyphénoxy)-*N*-[(3*S*)-2-oxotétrahydro-3-furanyl] acétamide
5-nitro-1-propylindoline ;
1-propyl-2,3-dihydro-1*H*-indol-5-ylamine;
3-oxo-2-phényl-N-(1-propyl-2,3-dihydro-1H-indol-5-yl)-béta-alanine ;
N¹-[(3S)-2-oxotétrahydro-3-furanyl]-2-phényl-N³-(1-propyl-2,3-dihydro-1H-indol-5-yl)malonamide;
*N*-(2-anilinophényl)-*N*'-[(3*S*)-2-oxotétrahydro-3-furanyl]urée;
oxo[(1-propyl-2,3-dihydro-1H-indol-5-yl)amino]acétate d'éthyle ;
acide oxo[(1-propyl-2,3-dihydro-1H-indol-5-yl)amino]acétique ;
(2S)-2-({[(2R)-6-hydroxy-2,5,7,8-tétramethyl-3,4-dihydro-2H-chromen-2-yl]carbonyl}amino)-3-phenylpropanoate de méthyle ;
(2R)-N-[(1S)-1-benzyl-2-oxoéthyl]-6-hydroxy-2,5,7,8-tétramethyl-3,4-dihydro-2H-chromène-2-carboxamide ;
5-methyl 1,5-indolinedicarboxylate de *tert*-butyle ;
acide 1-(*tert*-butoxycarbonyl)-5-indolinecarboxylique ;
5-({[(3S)-2-oxotétrahydro-3-furanyl]amino}carbonyl)-1-indolinecarboxylate de *tert-*butyle ;
5-({[(3S)-2-hydroxytétrahydro-3-furanyl]amino}carbonyl)-1-indolinecarboxylate de *tert*-butyle.

### Partie expérimentale

Les exemples suivants sont présentés pour illustrer les procédures ci-dessus et ne doivent en aucun cas être considérés comme une limite à la portée de l'invention.

### Exemple 17 : N-[(1S)-1-({[(3S)-2-hydroxytétrahydro-3-furanyl]amino}carbonyl)-3-méthylbutyl]-10H-phénothiazine-2-carboxamide :

### 17.1) (2S)-4-méthyl-2-[(10H-phénothiazin-2-ylcarbonyl)amino]pentanoate de méthyle :

A une solution de 1,82 g (10 mmoles) du chlorhydrate de l'ester méthylique de la L-Leucine, 2,43 g (10 mmoles) de l'acide 10H-phénothiazine-2-carboxylique (J. Med. Chem.(1998) 41 (2), 148-156), 1,48 g (11 mmoles) de HOBT et 4,21 g (22 mmoles) de EDC dans 30 ml de DMF anhydre, on ajoute 4,6 ml (33 mmoles) de triéthylamine. Le mélange réactionnel est agité pendant 15 heures. Après évaporation du solvant sous vide, le résidu est partitionné entre 100 ml d'AcOEt et 50 ml d'une solution 1M d'HCl. La phase organique est décantée et lavée successivement par 50 ml H₂O, 50 ml d'une solution saturée de NaHCO₃ et 50 ml de saumure. La solution organique est séchée sur sulfate de magnésium, filtrée et concentrée à sec sous vide. Le résidu d'évaporation est repris par Et₂O et filtré. Poudre jaune (71 %). Point de fusion : 160,5-161° C.

### 17.2) Acide (2S)-4-méthyl-2-[(10H-phénothiazin-2-ylcarbonyl)amino]pentanoïque :

A une solution de 1,85 g (5 mmoles) de l'intermédiaire 17.1 dans 20 ml de THF, on ajoute en une fois une solution de 0,44 g (11 mmoles) de LiOH, H₂O dans 20 ml d'H₂O. Le mélange réactionnel est agité 1 h 30 à 20° C. L'ensemble est refroidi à l'aide d'un bain de glace avant l'addition d'une solution aqueuse concentrée d'HCl jusqu'à pH acide. Après dilution par 100 ml d'AcOEt et agitation, la phase organique est décantée. Celle-ci est ensuite lavée par 20 ml d'une solution aqueuse 1M d'HCl suivi de 20 ml de saumure. La solution organique est séchée sur sulfate de sodium, filtrée et concentrée à sec sous vide. Poudre jaune-vert. Le produit est utilisé tel quel dans l'étape suivante.

### 17.3) N-[(1S)-3-méthyl-1-({[(3S)-2-oxotétrahydro-3-furanyl]amino}carbonyl)butyl]-10H-phénothiazine-2-carboxamide :

Le protocole expérimental utilisé est le même que celui décrit pour l'intermédiaire. 12.2, l'intermédiaire 17.2 remplaçant l'intermédiaire 12.1. Poudre jaune. Point de fusion : 151-152° C.

### 17.4) N-[(1S)-1-({[(3S)-2-hydroxytétrahydro-3-furanyl]amino}carbonyl)-3-méthyl butyl]-10H-phénothiazine-2-carboxamide :

Le protocole expérimental utilisé est le même que celui décrit pour l'intermédiaire 1.2, l'intermédiaire 17.3 remplaçant l'intermédiaire 1.1. Poudre jaune. Point de fusion : 100-101° C

### Exemple 18 : Acétate de (3S)-3-({(2S)-4-méthyl-2-[(10H-phénothiazin-2-ylcarbonyl)-amino]pentanoyl}amino)tétrahydro-2-furanyl :

Le protocole expérimental utilisé est le même que celui décrit pour l'exemple 13, à partir de l'intermédiaire 17.4. Les deux diastéréoisomères 18.1 et 18.2 sont séparés par chromatographie sur colonne de silice (éluant : Heptane/AcOEt : 1/1).

### 18.1) Acétate de (2R,3S)-3-({(2S)-4-méthyl-2-[(10H-phénothiazin-2-ylcarbonyl)amino]-pentanoyl}amino)tétrahydro-2-furanyl :

Poudre jaune pâle. Point de fusion : 199-201° C.

### 18.2) Acétate de (2S,3S)-3-({(2S)-4-méthyl-2-[(10H-phénothiazin-2-ylcarbonyl) amino]-pentanoyl}amino)tétrahydro-2-furanyl :

Poudre jaune pâle. Point de fusion : 205-208° C.

### Exemple 19 : N-[(3S)-2-hydroxytétrahydro-3-furanyl]-2-(10H-phénothiazin-2-yl)-1,3-thiazole-4-carboxamide :

### 19.1) 10H-phénothiazine-2-carbothioamide :

Un mélange réactionnel composé de 3,4 g (14 mmoles) de 10*H*-phénothiazine-2-carboxamide (J. Org. Chem. (1961) 26, 1138-1143) et de 3,4 g (8,4 mmoles) de réactif de Lawesson en solution dans 40 ml de 1,4-dioxanne additionné de 20 ml de pyridine est chauffé à 110° C pendant 1 h 30. La solution brune est ensuite concentrée sous vide et le résidu est dilué dans 200 ml d'AcOEt et 100 ml d'H₂O. Après agitation et décantation, la phase organique est lavée successivement par 100 ml d'une solution aqueuse 1N d'HCl et 100 ml de saumure. Après séchage sur sulfate de sodium, filtration et évaporation du solvant sous vide on obtient une poudre orange. Cette poudre est lavée par Et₂O, le filtrat est éliminé, et extraite par de l'acétone. Le filtrat acétonique est alors concentré sous vide et le résidu d'évaporation est alors purifié sur une colonne de silice (éluant : Heptane/AcOEt : 1/1 jusqu'à 4/6). Poudre orange. Point de fusion : 208-209° C.

### 19.2) 2-(10H-phénothiazin-2-yl)-1,3-thiazole-4-carboxylate d'éthyle :

A une suspension de 1,43 g (5,53 mmoles) de l'intermédiaire 19.1 dans 70 ml d'EtOH absolu, on ajoute 2,09 ml (16,5 mmoles) de bromopyruvate d'éthyle. Le mélange réactionnel est alors chauffé pendant 1 h 30 à reflux. Après concentration à sec sous vide, le résidu noir obtenu est lavé par Et₂O avant d'être déposé au sommet d'une colonne de chromatographie (élution : Heptane/AcOEt/THF : 6/4/0 jusqu'à THF pur). Poudre jaune (83 %).

### 19.3) Acide 2-(10H-phénothiazin-2-yl)-1,3-thiazole-4-carboxylique :

Une solution de l'intermédiaire 19.2 (1,62 g, 4,57 mmoles) dans 50 ml de THF est refroidie à 0° C avant l'addition en une portion d'une solution de 300 mg (7,3 mmoles) de NaOH dans 30 ml H₂O. L'agitation est poursuivie 15 heures à 20° C avant d'acidifier le mélange réactionnel, à 0° C, par une solution aqueuse d'HCl concentrée. Le produit est alors extrait à l'aide de 100 ml d'AcOEt et la solution organique est lavée par 25 ml d'H₂O suivi de saumure. Après séchage sur sulfate de sodium, filtration et concentration sous vide, le résidu est purifié sur une colonne de silice (éluant : CH₂Cl₂/MeOH: 8/2 jusqu'à 1/1). Poudre jaune.

### 19.4) N-[(3S)-2-oxotétrahydro-3-furanyl]-2-(10H-phénothiazin-2-yl)-1,3-thiazole-4-carboxamide :

Le protocole expérimental utilisé est le même que celui décrit pour l'intermédiaire 12.2, l'intermédiaire 19.3 remplaçant l'intermédiaire 12.1. Poudre jaune. Point de fusion : 277-277,5° C.

### 19.5) N-[(3S)-2-hydroxytétrahydro-3-furanyl]-2-(10H-phénothiazin-2-yl)-1,3-thiazole-4-carboxamide :

Le protocole expérimental utilisé est le même que celui décrit pour l'intermédiaire 1.2, l'intermédiaire 19.4 remplaçant l'intermédiaire 1.1. Poudre jaune. Point de fusion : 189-190° C.

### Exemple 20: N-[4-({[(3S)-2-hydroxytétrahydro-3-furanyl]amino}carbonyl)phényl]-10H - phénothiazine-2-carboxamide :

### 20.1) Acide 4-[(10H-phénothiazin-2-ylcarbonyl)amino]benzoïque :

Le protocole expérimental utilisé est le même que celui décrit pour les synthèses des intermédiaires 17.1 et 17.2, l'acide 4-aminobenzoate de méthyle remplaçant l'ester méthylique de la L-Leucine.

### 20.2) N-[4-({[(3S)-2-oxotétrahydro-3-furanyl]amino}carbonyl)phényl]-10H-phénothiazine -2-carboxamide :

Le protocole expérimental utilisé est le même que celui décrit pour la synthèse de l'intermédiaire 12.2, l'intermédiaire 20.1 remplaçant l'acide 4-(4-anilinoanilino)-4-oxobutanoïque. Poudre jaune-vert. Point de fusion : 284-285° C.

### 20.3) N-[4-({[(3S)-2-hydroxytétrahydro-3-furanyl]amino}carbonyl)phényl]-10H-phénothiazine-2-carboxamide :

Le protocole expérimental utilisé est le même que celui décrit pour l'intermédiaire 1.2, l'intermédiaire 20.2 remplaçant l'intermédiaire 1.1. Poudre jaune foncée. Point de fusion : 234-235° C.

### Exemple 21 : N-[(1S)-1-({[(3S)-2-hydroxytetrahydro-3-furanyl]amino}carbonyl)-3-methylbutyl]-10H-phenothiazine-1-carboxamide :

Le protocole expérimental utilisé est identique à celui décrit pour le composé 17, l'acide 10*H*-phénothiazine-1-carboxylique remplaçant l'acide 10*H*-phénothiazine-2-carboxylique. Poudre jaune. Point de fusion : 99-101° C.

### Exemple 22 : Pivalate de (3S)-3-({(2S)-4-méthyl-2-[(10H-phénothiazin-2-ylcarbonyl) amino]pentanoyl}amino)tétrahydro-2-furanyl :

On additionne goutte-à goutte 0,14 ml de chlorure de 2,2-dimethylpropanoyle à une solution de 0,45 g (1,02 mmole) de l'intermédiaire 17.4 et de 0,28 ml (2,04 mmoles) de Et₃N dans 20 ml de CH₂Cl₂ refroidie à 0°C. Le mélange réactionnel est ensuite agité 24 heures à 22°C. Après dilution par 50 ml de CH₂Cl₂, la solution organique est lavée par 20 ml d'eau suivi de 20 ml de saumure, séchée sur MgSO₄, filtrée et concentrée à sec sous vide. Le produit est finalement purifié par chromatographie sur une colonne de silice (éluant : Heptane/AcOEt : 1/1). Solide jaune pâle. Point de fusion : 107-109°C.

### Exemple 23 : 3,3-diméthylbutanoate de (3S)-3-({(2S)-4-méthyl-2-[(10H-phénothiazin-2-ylcarbonyl)amino]pentanoyl }amino)tétrahydro-2-furanyl :

Le protocole expérimental utilisé est le même que celui décrit pour l'exemple 22, à partir de l'intermédiaire 17.4 et du chlorure de 3,3-dimethylbutanoyle. Solide jaune. Point de fusion : 111-113°C.

### Exemple 24 : (3S)-3-({(2S)-4-méthyl-2-[(10H-phénothiazin-2-ylcarbonyl)amino] pentanoyl }amino)tétrahydro-2-furanyl benzoate :

Le protocole expérimental utilisé est le même que celui décrit pour l'exemple 22, à partir de l'intermédiaire 17.4 et du chlorure de benzoyle. Solide jaune pâle. Point de fusion : 193-195°C.

### Exemple 25 : (3S)-3-({(2S)-4-méthyl-2-[(10H-phénothiazin-2-ylcarbonyl)amino] pentanoyl}amino)tétrahydro-2-furanyl phénylacetate :

Le protocole expérimental utilisé est le même que celui décrit pour l'exemple 22, à partir de l'intermédiaire 17.4 et du chlorure de phénylacétyle. Solide jaune. LC-MS : MH⁺ = 560,2.

### Exemple 26 : (3S)-3-({(2S)-4-méthyl-2-[(10H-phénothiazin-2-ylcarbonyl)amino] pentanoyl}amino)tétrahydro-2-furanyl (2S)-2-(diméthylamino)-3-phénylpropanoate :

A une solution de 0,5 g (1,13 mmole) de l'intermédiaire 17.4 dans 2 ml de CH₂Cl₂, on ajoute 0,22 g (1,13 mmole) de l'acide (2*S*)-2-(diméthylamino)-3-phénylpropanoïque et 0,23 g (1,13 mmole) de 1,3-dicylohexylcarbodiimide. Après 72 h d'agitation à 22°C, le précipité est filtré et lavé par 10 ml de CH₂Cl₂. Le filtrat est ensuite lavé par une solution saturée de NaHCO₃ (10 ml) suivi de 10 ml d'eau et 10 ml de saumure. La solution organique est séchée sur MgSO₄, filtrée et concentrée à sec. Le résidu d'évaporation est purifié sur une colonne de silice (éluant : AcOEt). Solide jaune. LC-MS : MH⁺ = 617,2.

### Exemple 27 : 4-morpholinecarboxylate de (3S)-3-({(2S)-4-méthyl-2-[(10H-phénothiazin-2-ylcarbonyl)amino]pentanoyl}amino)tétrahydro-2-furanyl:

Le protocole expérimental utilisé est le même que celui décrit pour l'exemple 22, à partir de l'intermédiaire 17.4 et de chloroformiate de morpholine. Solide jaune. Point de fusion : 165-167°C.

### Etude pharmacologique des produits de l'invention

### Etude des effets sur la Calpaine I porcine :

La méthode utilisée est celle décrite par Mallya et coll. (Biochemical and biophysical research communications 248 293-296 (1998). L'activité calpaia *in vitro* est suivie par la mesure de la fluorescence due à la dégradation d'un substrat artificiel de l'enzyme Suc-LY-AMC (Suc-Leu-Tyr-aminométhylcoumarine). L'aminométhylcoumarine libérée fluoresce à 460 nm sous une excitation à 380 nm. Les inhibiteurs testés sont dissous dans du DMSO à 40 fois la concentration finale. 5 µl de solution sont déposés dans une plaque 96 puits à paroi noire. 175 µl/puits de tampon de réaction contenant la calpain 1 et son substrat sont alors ajoutés. La réaction est démarrée par l'ajout de 20 µl de CaCl₂ 50 mM. Les plaques sont incubées à 25° C et la fluorescence (380 nm excitation et 460 nm émission) est lue au bout de 30 minutes à l'aide d'un lecteur de microplaques (Victor, Wallack). Les CI₅₀ sont déterminées par le calcul des rapports fluorescence produit /fluorescence témoin DMSO. Composition du tampon de la réaction enzymatique : Tris-HCl 50 mM pH 7.5, NaCl 50 mM, EDTA 1 mM, EGTA 1 mM, b-Mercaptoéthanol 5 mM, Suc-LY-AMC 1mM (Bachem, ref. I-1355) et 2.5 U/ml Calpain I (érythrocytes porcins, Calbiochem ref 208712). ). Sur ce test, la valeur IC₅₀ de certains composés selon l'invention est inférieure à 5µM.

### Etude des effets sur la péroxidation lipidique du cortex cérébral de rat :

L'activité inhibitrice des produits de l'invention est déterminée par la mesure de leurs effets sur le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, *Meth. Enzymol*. (1990) 186 : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50 000 g pendant 10 minutes à 4° C. Le culot est conservé à - 80° C. Le jour de l'expérience, le culot est remis en suspension à la concentration de 1 g /15 ml et centrifugé à 515 g pendant 10 minutes à 4°C. Le surnageant est utilisé immédiatement pour la détermination de la péroxidation lipidique. L'homogénat de cortex cérébral de rat (500 µl) est incubé à 37° C pendant 15 minutes en présence des composés à tester ou du solvant (10 µl). La réaction de péroxidation lipidique est initiée par l'ajout de 50 µl de FeCl₂ à 1 mM, d'EDTA à 1 mM et d'acide ascorbique à 4 mM. Après 30 minutes d'incubation à 37° C, la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45° C. La condensation d'une molécule de MDA avec deux molécules de réactif R produit un chromophore stable dont la longueur d'onde d'absorbance maximale est égale à 586 nm. (Caldwell et coll. European J. Pharmacol. (1995) 285, 203-206). Sur ce test, la valeur IC₅₀ des composés selon l'invention est inférieure à 5 µM.

## Revendications

1. Composés de formule générale (I) 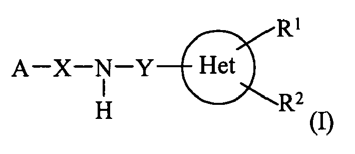 sous forme racémique, d'énantiomères, de diastéréoisomères ou toutes combinaisons de ces formes, dans laquelle
R¹représente l'atome d'hydrogène, le radical -OR³ ou oxo,
dans lequel R³ représente un atome d'hydrogène, un radical alkyle, arylalkyle, hétérocycloalkylcarbonyle, alkylcarbonyle, arylcarbonyle ou aralkylcarbonyle,
dans lesquels les radicaux alkyle, aryle ou hétérocycloalkyle sont éventuellement substitués par un ou plusieurs substituants identiques ou différents choisis parmi :
alkyle, OH, alkoxy, nitro, cyano, halogène ou -NR⁴R⁵ ;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
R²représente un atome d'hydrogène ou un radical aralkyle,
A représente un radical A4 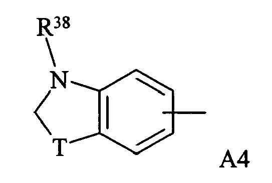 dans lequel R³⁸ représente un atome d'hydrogène, un radical alkyle, -(CH₂)_{q}-NR³⁹R⁴⁰ ou aralkyle, le groupement aryle étant éventuellement substitué par un ou plusieurs substituants identiques ou différents choisis parmi : OH, alkyle, halogène, nitro, alkoxy ou -NR³⁹R⁴⁰,
q étant un entier compris entre 2 et 6 ;
R³⁹ et R⁴⁰ représentent, indépendamment, un atome d'hydrogène, un radical alkyle ou un groupe -COR⁴¹, ou bien R³⁹ et R⁴⁰ forment ensemble avec l'atome d'azote un hétérocycle éventuellement substitué,
R⁴¹ représente un atome d'hydrogène, un radical alkyle, alkoxy ou -NR⁴²R⁴³,
R⁴² et R⁴³ représentent, indépendamment, un atome d'hydrogène ou un radical alkyle, ou bien R⁴² et R⁴³ forment ensemble avec l'atome d'azote auquel ils sont rattachés un hétérocycle éventuellement substitué,
T représentant le radical -(CH₂)- ,
X représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Y représente -(CH₂)ₚ- ,
p étant un entier compris entre 0 et 6 ;
Het représente l'hétérocycle tétrahydrofuranne, dioxolane, pyrrolidine ou 1,3-oxazolidine,
ainsi que les sels d'addition avec les acides minéraux et organiques ou avec les bases minérales et organiques desdits composés de formule générale (I),
à l'exclusion des composés de formule (I) dans laquelle lorsque Het représente tétrahydrofuranne ou tétrahydropyranne, R¹ le radical OR³ avec R³ représentant un atome d'hydrogène, un radical alkyle, arylalkyle, hétérocycloalkylcarbonyle dont le radical hétérocycloalkyle est branché par un atome de carbone, alkylcarbonyle, arylcarbonyle ou aralkylcarbonyle, R² un hydrogène et Y le radical -(CH₂)ₚ- avec p = 0, alors X représente -CO-N(R⁴⁵)-C(R⁴⁶R⁴⁷)-CO- avec R⁴⁵ = R⁴⁶ = H.

2. Composés selon l'une des revendications précédentes, **caractérisés en ce** X représente -(CH₂)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO ou -Z-CO-.

3. Composés selon la revendication 2, **caractérisés en ce que** R⁴⁵ et R⁴⁷ représentent l'atome d'hydrogène, R⁴⁶ l'atome d'hydrogène, un radical alkyle ou phényle, D le radical phénylène et Z le radical thiazole.

4. Composés selon l'une des revendications précédentes, **caractérisés en ce que** le radical aralkyle que représente R² est le radical benzyle.

5. Composés répondant à l'une des formules suivantes :
N¹-[(3S)-2-hydroxytétrahydro-3-furanyl]-2-phényl-N³-(1-propyl-2,3-dihydro-1H-indol-5-yl)malonamide ;
N¹-[(3S)-2-hydroxytetrahydro-3-furanyl]-N²-(1-propyl-2,3-dihydro-1H-indol-5-yl) ethanediamide ;
N-[(3S)-2-hydroxytétrahydro-3-furanyl]-5-indolinecarboxamide.
(2S)-2-({[(1-benzyl-2,3-dihydro-1H-indol-5-yl)amino]carbonyl}amino)-N-[(3S)-2-hydroxytétrahydro-3-furanyl]-4-méthylpentanamide ;
(2S)-N-[(3S)-2-hydroxytétrahydro-3-furanyl]-4-méthyl-2-[({[1-(1-naphtylméthyl)-2,3-dihydro-1H-indol-5-yl]amino}carbonyl)amino]pentanamide.

6. A titre de médicaments, des composés selon l'une des revendications 1 à 5.

7. Compositions pharmaceutiques comprenant, à titre de principe actif, au moins un médicament tel que défini à la revendication 6.

8. Utilisation de composés de formule (Iₐ) telle que définie ci-dessus, 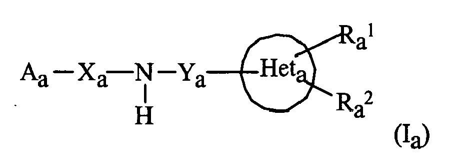 sous forme racémique, d'énantiomères, de diastéréoisomères ou toutes combinaisons de ces formes, dans laquelle
Rₐ¹représente l'atome d'hydrogène, le radical -OR³ ou oxo,
dans lequel R³ représente un atome d'hydrogène, un radical alkyle, arylalkyle, hétérocycloalkylcarbonyle, alkylcarbonyle, arylcarbonyle ou aralkylcarbonyle,
dans lesquels les radicaux alkyle, aryle ou hétérocycloalkyle sont éventuellement substitués par un ou plusieurs substituants identiques ou différents choisis parmi :
alkyle, OH, alkoxy, nitro, cyano, halogène ou -NR⁴R⁵ ;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
Rₐ²représente un atome d'hydrogène ou un radical aralkyle,
Aₐreprésente un radical A4 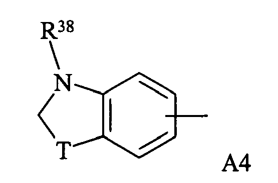 dans lequel R³⁸ représente un atome d'hydrogène, un radical alkyle, -(CH₂)_{q}-NR³⁹R⁴⁰ ou aralkyle, le groupement aryle étant éventuellement substitué par un ou plusieurs substituants identiques ou différents choisis parmi : OH, alkyle, halogène, nitro, alkoxy ou -NR³⁹R⁴⁰,
q étant un entier compris entre 2 et 6 ;
R³⁹ et R⁴⁰ représentent, indépendamment, un atome d'hydrogène, un radical alkyle ou un groupe -COR⁴¹, ou bien R³⁹ et R⁴⁰ forment ensemble avec l'atome d'azote un hétérocycle éventuellement substitué,
R⁴¹ représente un atome d'hydrogène, un radical alkyle, alkoxy ou -NR⁴²R⁴³,
R⁴² et R⁴³ représentent, indépendamment, un atome d'hydrogène ou un radical alkyle, ou bien R⁴² et R⁴³ forment ensemble avec l'atome d'azote auquel ils sont rattachés un hétérocycle éventuellement substitué,
T représentant le radical -(CH₂)-,
Xₐ représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Yₐ représente -(CH₂)ₚ- ;
p étant un entier compris entre 0 et 6 ;
Hetₐ représente l'hétérocycle tétrahydrofuranne, dioxolane, pyrrolidine ou 1,3-oxazolidine,
ainsi que des sels d'addition avec les acides minéraux et organiques ou avec les bases minérales et organiques desdits composés de formule générale (I),
pour la préparation de médicaments pour le traitement de pathologies où les calpaïnes et / ou les formes réactives de l'oxygène sont impliquées.

9. Utilisation de composés de formule (Iₐ) selon la revendication 8, pour la préparation de médicaments pour le traitement de pathologies où les formes réactives de l'oxygène sont impliquées.

10. Utilisation de composés de formule (Iₐ) selon la revendication 8, pour la préparation de médicaments pour le traitement de pathologies où les formes réactives de l'oxygène et les calpaïnes sont impliquées.

11. Utilisation de composés de formule (Iₐ) selon l'une des revendications 8 à 10, pour la préparation de médicaments pour le traitement de pathologies des maladies inflammatoires et immunologiques, des maladies cardio-vasculaires et cérébro-vasculaires, des troubles du système nerveux central ou phériphérique, de l'ostéoporose, des dystrophies musculaires, des maladies prolifératives, de la cataracte, des transplantations d'organes, des maladies auto-immunes et virales, du cancer, et de toutes les pathologies **caractérisées par** une production excessive des ROS et / ou une activation des calpaïnes.

12. Utilisation de composés de formule (Iₐ) selon l'une des revendications 8 à 11, **caractérisés en ce que** X représente -(CH₂)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO ou -Z-CO-.

13. Utilisation de composés de formule (Iₐ) selon la revendication 12, **caractérisés en ce que** R⁴⁵ et R⁴⁷ représentent l'atome d'hydrogène, R⁴⁶ l'atome d'hydrogène, un radical alkyle ou phényle, D le radical phénylène et Z le radical thiazole.

14. Utilisation de composés de formule (Iₐ) selon l'une des revendications 8 à 13, **caractérisés en ce que** le radical aralkyle que représente Rₐ²est le radical benzyle.

15. Utilisation de composés de formule (Iₐ) selon l'une des revendications 8 à 14, **caractérisés en ce qu'**ils répondent à l'une des formules suivantes :
N¹-[(3S)-2-hydroxytétrahydro-3-furanyl]-2-phényl-N³-(1-propyl-2,3-dihydro-1H-indol-5-yl)malonamide ;
N¹-[(3S)-2-hydroxytetrahydro-3-furanyl]-N²-(1-propyl-2,3-dihydro-1H-indol-5-yl) ethanediamide ;
N-[(3S)-2-hydroxytétrahydro-3-furanyl]-5-indolinecarboxamide,
(2S)-2-({[(1-benzyl-2,3-dihydro-1H-indol-5-yl)amino]carbonyl}amino)-N-[(3S)-2-hydroxytetrahydro-3-furanyl]-4-methylpentanamide ;
(2S)-N-[(3S)-2-hydroxytetrahydro-3-furanyl]-4-methyl-2-[({[1-(1-naphthhylmethyl)-2,3-dihydro-1H-indol-5-yl]amino}carbonyl)amino]pentanamide.
